# EUROPEAN PATENT APPLICATION

(11) **EP 1 688 095 A1**
(43) Date of publication of application: **09.08.2006**
(21) Application number: 04792945.0
(22) Date of filing: 26.10.2004
(51) Int. Cl.: A61B 17/04

(54) **SUTURE THREAD SHIFTING DEVICE**

(30) Priority: 28.10.2003 JP 2003367292
(71) Applicant: Nihon University, Tokyo 102-8275 (JP)
(72) Inventor: HORAGUCHI, Takashi, Nihon University, Tokyo 1028275 (JP)
(74) Representative: HOFFMANN EITLE
(86) International application number: PCT/JP2004/015817
(87) International publication number: WO 2005/039420

(57) **Abstract**

The suture thread shifting device is **characterized in that** even when the incision portion of surface layer of the body is small, front edge (11) of the device has a sectional configuration (for example, circular section of relatively small diameter) suitable for allowing the front edge to reach a region in arthroscopic or endoscopic viewing field and that the device with the suture thread engaging in the front edge (11) can shift the suture thread from a position on the operating surgeon's side (near side position)to a position remote from the operating surgeon (far side position).

## Description

### Technical Field

The present invention relates to a device for shifting a position of a suture thread at the surgery conducted in arthroscopic or endoscopic viewing field.

### Background Art

For example, when bone dislocation of a joint portion is treated, a hole having a diameter of about 1 cm is formed in a skin of a diseased portion, and an arthroscopic or endoscopic camera is inserted into a human body through the hole. In addition, an image photographed, for example, by the endoscopic camera is enlarged and displayed on a monitor. In other words, the inside of the joint portion is enlarged and displayed on the monitor. In addition, in order to insert a surgery device at the surgery, two or three additional holes are formed (incised).

An arthroscopic or endoscopic camera has come into wide use as an effective tool for the above-described treatment/surgery.
The diseased portion such as the dislocation of the joint portion is far back from the surface at the treatment/surgery. Although a portion close to the surface (skin) is not diseased, the portion must be significantly incised to approach the diseased portion (which is far back from the surface). However, when the portion is significantly incised, it takes much time to restore a muscle force. In addition, a wound may be opened. Accordingly, a portion which is not diseased (such as skin or muscle) is requested to be not incised.
For such a request, arthroscope and endoscope can be effectively used. Thus, the arthroscope and the endoscope have come into wide use.
By using the arthroscope or the endoscope, it is possible to avoid unnecessarily inserting a surgeon's knife into the portion which is not diseased and to perform a surgery without significant incision (into the skin or the muscle which is not diseased).

Meanwhile, when joint surgery is conducted in arthroscopic or endoscopic viewing field, the surgery is conducted using a small tool or device. Thus, the surgery itself has become complicated.
For example, at the surgery for shoulder dislocation, as shown in surgery processes of Figs. 9 to 11, an anchor is inserted into a shoulder blade (Fig. 9) and a suture thread passes through an articular labrum and an articular capsule (Figs. 10 and 11), thereby accomplishing suture (Fig. 11). Here, problems are generated
(a) when the suture thread falls away from the anchor,
(b) when the suture thread exists at a portion different from an expected portion, and
(c) when a surgery device interferes with a bone and thus cannot be operated as intended.

Conventionally, a device for shifting a suture thread from a side more remote from an operating surgeon (far side) than an expected position to a position on the operating surgeon's side (near side), for example, a hook-shaped device or a pinch type device, is disclosed (For example, see Non-Patent Document 1).

However, a dedicated device for shifting a suture thread from a side nearer to the operating surgeon than the expected position to the far side has not existed.

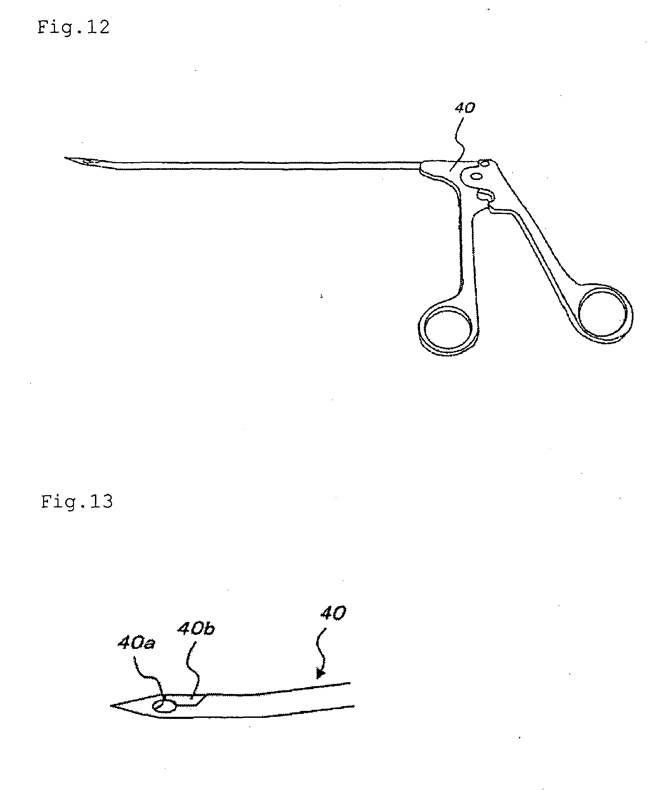

A device for gripping and shifting a thread (pinch type device; see Figs. 12 to 14) can shift a suture thread from the operating surgeon's near side to the operating surgeon's far side.

However, in the pinch type device, since the volume of the device for gripping the thread (clamp portion) is large, when tucking the suture thread (from the operating surgeon's near side to the operating surgeon's far side), the device is met with resistance and thus human body tissue may be injured.
In addition, in a portion in which a plurality of suture threads exists, there is no space for the clamp portion of the pinch type device to be moved, and the other suture thread (which must not be moved) may be moved.
Accordingly, it is difficult for an unskilled person to tuck the suture thread from the operating surgeon's near side to the operating surgeon's far side using the pinch type device.
Non-patent Document 1: "The Complete System for SHOULDER ARTHROSCOPY," Medical Instrument Catalog of T.A.G. Corporation, published by CBC Corporation, February, 2002.

### Disclosure of the Invention

### Problem to be Solved by the Invention

The present invention is contrived to solve the above-described problems of the related art, and an object of the present invention is to provide a suture thread shifting device with which, at the surgery conducted in arthroscopic or endoscopic viewing field, operating surgeon even if not highly skilled can accurately and easily shift a fibrous member, such as suture thread, from a position on the operating surgeon's near side to a position on the far side without interference with human body tissue.

### Means for Solving the Problem

The suture thread shifting device (1) according to the present invention is characterized in that even when the incision portion (0) of surface layer of the body is small, front edge (11) of the device has a sectional configuration (for example, circular section of relatively small diameter) suitable for allowing the front edge (11) to reach a region in arthroscopic or endoscopic viewing field and that the device with the suture thread engaging in the front edge (11) can shift the suture thread from a position on the operating surgeon's side (near side position) to a position remote from the operating surgeon (far side position). (Claim 1)

It is preferable that the front edge (11) is bisected, the lengths (L1 and L2) of bisected portions are different from each other (that is, bilaterally asymmetrical), and the surfaces of the bisected portions (11) are smooth (Claim 9).

In addition, when the shoulder dislocation is treated using the suture thread shifting device (1) according to the present invention, the surface layer near the shoulder joint (K) is incised (O) with a relatively small size, arthroscope or endoscope is inserted through the incision portion (O) to observe a portion at which a cartilage (21) and an articular labrum (22) are stripped, anchor implants (4) are inserted into a plurality of portions of a shoulder blade (2), the suture thread (5) passes through the articular capsule (23) and the articular labrum (22), the suture thread (5), which passes through the articular capsule (23) and the articular labrum (22), are coupled to the anchor implants (4), the suture thread shifting device (1) is inserted into the human body through the incision portion (0) having the relatively small diameter of the surface layer of the human body when the suture thread (5) has come to the position on the operating surgeon's nearer side than a predetermined position, and the suture thread (5) is engaged with the front edge (11) of the suture thread shifting device (1) and shifted from the position on the operating surgeon's side (near side position) to the position remote from the operating surgeon (far side position).

### Effect of the Invention

According to the suture thread shifting device (1) of the present invention having such a configuration, the suture thread (5) is engaged with the front edge (11) of the device and the front edge (11) of the suture thread shifting device (1) is shifted to a predetermined position remote from the operating surgeon (far side). Thus, it is possible to accurately and easily shift the suture thread (5) from the position on the operating surgeon's side (near side position) to the position remote from the operating surgeon (far side position).

In addition, the front edge (11) is bisected and the lengths (L1 and L2) of the bisected portions (11) are different from each other. In other words, since the bisected portions (11) are bilaterally asymmetrical, the bisected portions can catch (wind) or disengage the suture thread (5) (release the wound thread) by rotating the suture thread shifting device (1). Thus, the usability of the suture thread shifting device (1) is excellent.

According to the present invention, a time for shifting the suture thread 5 from the position on the operating surgeon's side (near side position) to the position remote from the operating surgeon (far side position) shortens to about a half.

### Best Mode for carrying out the Invention ,

Hereinafter, embodiments according to the present invention will be described with reference to the accompanying drawings.
In the embodiments shown in the figures, the present invention is applied to surgery for shoulder dislocation, such as treatment for repetitive dislocation.

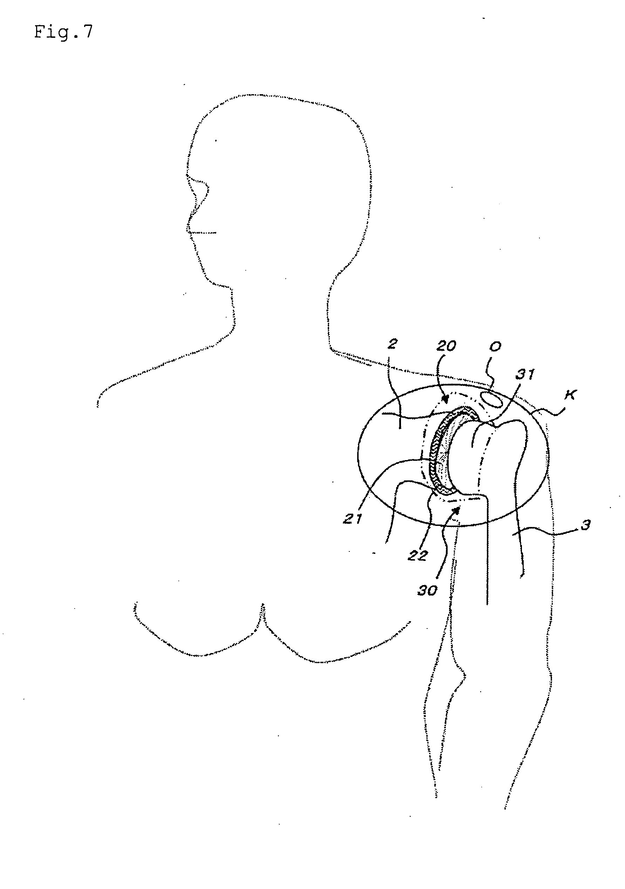

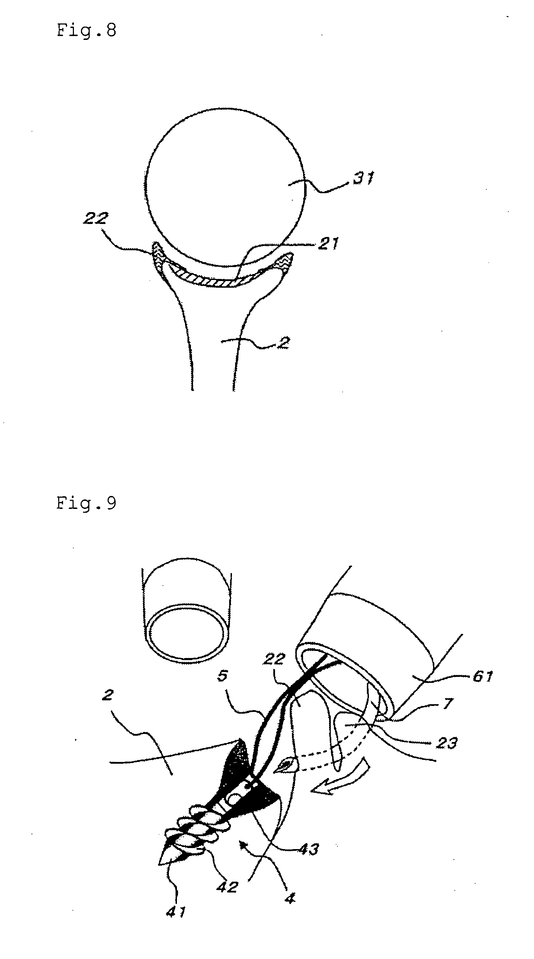

As shown in Fig. 7, a shoulder joint K is configured by mounting a spherical protrusion 31 of an end 30 of a humerus 3 on a golf-tee shaped end 20 of a shoulder blade 2, which is recessed in a spherical shape (similar to, for example, at a state where a golf ball is mounted on a golf tee; see Fig. 8). Accordingly, the movable range of the shoulder joint K is wide.

Since the golf-tee shaped end 20 has a shallow concave portion, the movable range of the spherical protrusion 31 of the end of the humerus 3 is wide.
In addition, the surface of the concave portion of the golf-tee shaped end 20 is made of cartilage 21, and articular labrum 22 made of fiber is connected to the cartilage 21 at the circumference of the concave portion 20 made of the cartilage 21.

When the shoulder joint K is dislocated, the articular labrum 22 is injured (stripped from the cartilage 21), and the spherical protrusion 31 falls away from the golf-tee shaped end 20. This is similar to a state where the golf ball drops from a golf tee.
When the articular labrum 22 is partially injured, the spherical protrusion 31 is apt to fall away from the golf-tee shaped end 20, as if a golf ball is apt to drop from a partially chipped golf tee. In other words, when the articular labrum 22 is injured, the spherical protrusion 31 is apt to fall away from the golf-tee shaped end 20 and thus the repetitive dislocation is apt to be caused.

The articular labrum 22 is generally broken up to about 165° of 360° (approximately, a half of a circumference) of the concave portion of the end 20 of the shoulder blade 2 (concave portion of the golf-tee shaped end: φ 3 cm) upon the dislocation (injury of articular labrum).

The articular labrum (fiber) 22 and the bone 2 are adhered in the order of the bone 2, the cartilage 21, and the articular labrum (fiber) 22. In addition, since the cartilage 21 and the articular labrum 22 are made of cells which are alternately adhered to one another in juxtaposition, the cartilage 21 and the articular labrum 22 are easily stripped.

An articular capsule 23 and the articular labrum 22 need be attached to the shoulder blade together in the injury of the articular labrum 22.

Hereinafter, the steps of a surgery for the shoulder dislocation as described above will be described with reference to Figs. 9 to 11.

Before conducting the surgery of Figs. 9 to 11, a small hole (incision portion) O for inserting a device used for the surgery into a surface layer is first formed in the vicinity of a diseased portion as shown in Fig. 7.

In addition, in Fig. 9, an anchor 4 is inserted into the shoulder blade 2 near the stripped articular labrum 22.
The anchor 4 has a sharp front end 41 having a conical shape, a screw portion 42 is formed from a cylindrical portion at which the cone of the front end 41 ends, and an elliptical hole 43 into which a suture thread 5 is inserted is formed at the rear end of the cylindrical portion.
In addition, the suture thread 5 is inserted into the elliptical hole 43 in advance.
Next, in the example shown in the figures, a "blitz suture retriever" 7, which is a tubular surgery needle having a curved front edge and passes through a first capsule-shaped body, that is, a "first cannula" 61 (is inserted into the first cannula 61), simultaneously passes through the articular capsule 23 and the articular labrum 22 from the side of the articular 23.

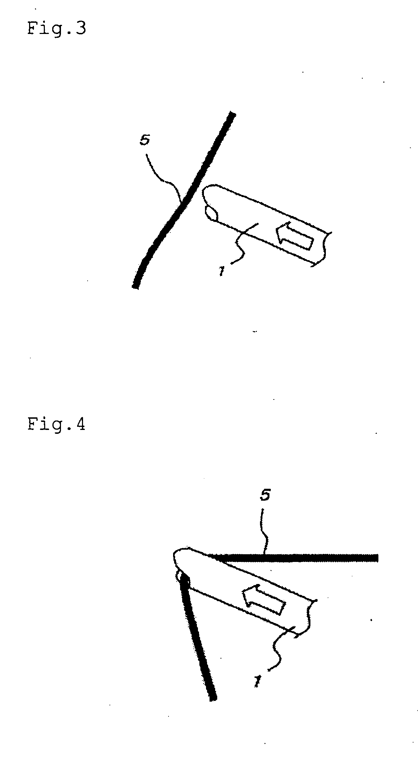

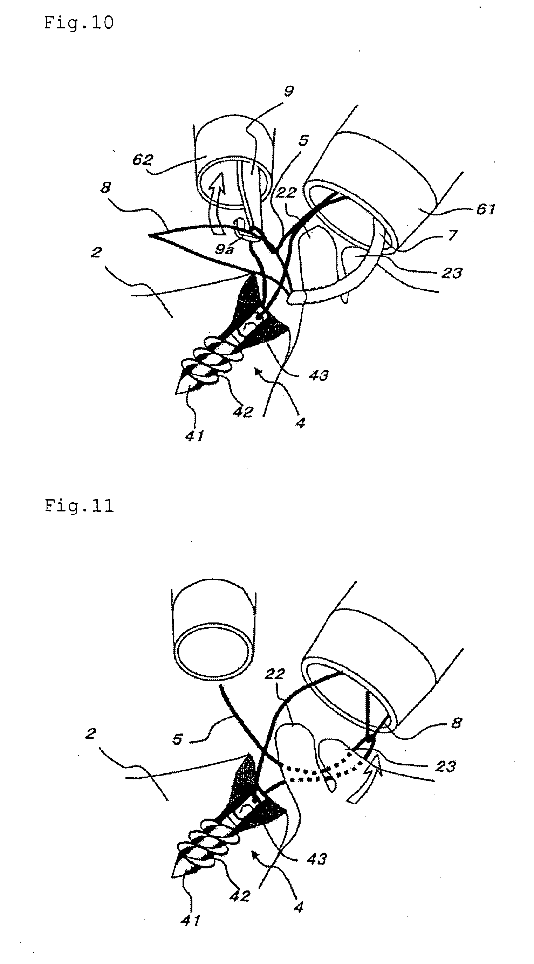

Next, in Fig. 10, in a state where the blitz suture retriever 7 simultaneously passes through the articular capsule 23 and the articular labrum 22, a wire loop 8 passes through the blitz suture retriever 7 such that the wire loop 8 gets out of the front edge of the blitz suture retriever 7.
The wire loop 8, which gets out of the front edge of the blitz suture retriever 7, expands at the rear side of the suture thread 5 (rear side of Fig. 3) in the joint using a device (not shown).
Next, a hook portion 9a of a crochet hook 9, which passes through a second cannula 62, is inserted into the wire loop 8 at the rear side of the wire loop 8 and protrudes to the front side of the figure, and the hook portion 9a is hitched to the suture thread 5. In a state where the crochet hook 9 is hitched to the suture thread 5, the crochet hook 9 returns to the second cannula 62.

Next, as shown in Fig. 11, in a state where the crochet hook 9 returns to the second cannula 62 (crochet hook 9 is not shown), the wire loop 8 is pushed (into the first cannula 61) by the blitz suture retriever 7 (not shown in Fig. 11) by pulling the blitz suture retriever 7 out of the articular labrum 22 and the articular capsule 23. Hereby, the suture thread 5 is inserted into (passes through) the articular capsule 23 and the articular labrum 22, enabling suture.

The anchors 4 are generally inserted into three or four points (about six points in special surgery) and connected to one another by the suture threads 5. The above-described treatment is continuously performed using the respective blitz suture retrievers 7.

After the surgery shown Figs. 9 to 11, tissues are adhered to one another to be reproduced. Even if the reproduction is insufficient (worst), the recurrence of the dislocation can be prevented since the tissues are fixed to one another by the suture thread 5.

When the suture thread 5 falls away from the anchor 4 at the surgery, that is, when the suture thread 5 is not located at an expected position, if, for example, the suture thread 5 is located at a position (far side position) more remote from an operating surgeon than the expected position, the suture thread is shifted to the operating surgeon's side (near side) using a conventional device, such as a pinch type device or a hook-shaped device, as described below.

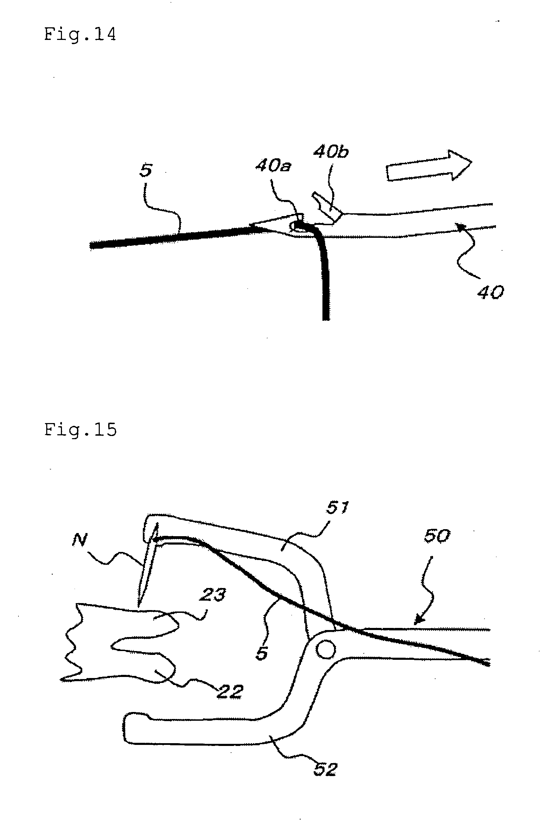

In other words, as shown in Fig. 14, the suture thread 5 is engaged with a suture-thread engagement hole 40a in the vicinity of the front edge of a pinch type suture inserting device 40 the entire body of which is shown in Fig. 12, and pulled to the operating surgeon's side (the front side of Figs. 9 to 11 or a direction indicated by an arrow in Fig. 14).
Fig. 13 shows a state where the suture-thread engagement hole 40a is formed such that a portion thereof opens and closes and an openable member 40b is closed.

As shown in Figs. 15 to 19, a method of suturing the articular labrum 22 and the articular capsule 23 with the suture thread 5 using a movable hook type suture inserting device 50 and engaging the articular labrum 22 and the articular capsule 23 with the anchor 4 may be employed.
In other words, in Fig. 15, a suture needle N is positioned at a predetermined position above the articular capsule 23 while grasping the suture needle N engaged with the suture thread 5 using a movable hook 51 of the movable hook type suture inserting device 50 having the movable hook 51 and a fixed hook 52.

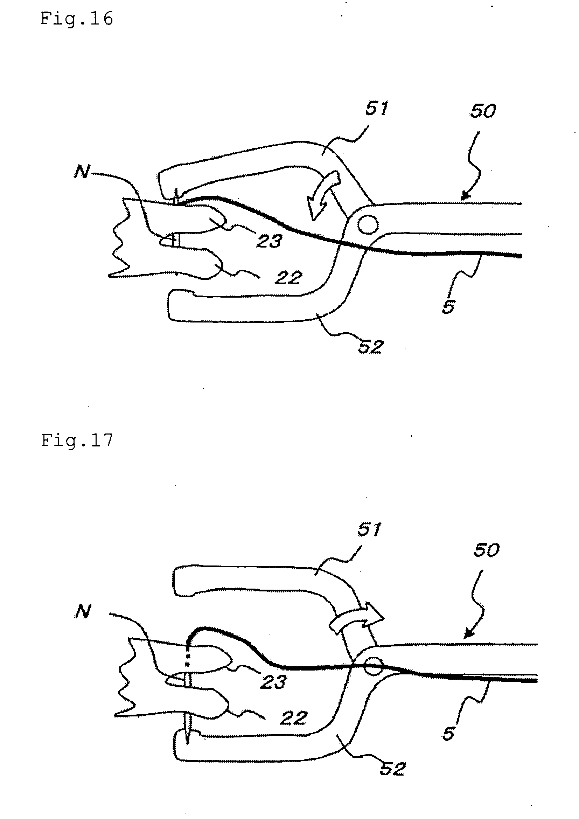

In Fig. 16, the movable hook 51 is moved to a closed side (direction indicated by an arrow) and the suture needle N pierces through the articular labrum 22 from the side of the articular capsule 23.

In Fig. 17, the suture needle N is deeply pushed into the articular capsule 23 and the articular labrum 22 again, the movable hook 51 is then separated from the suture needle N, and then the movable hook 51 opens in a direction indicated by an arrow.

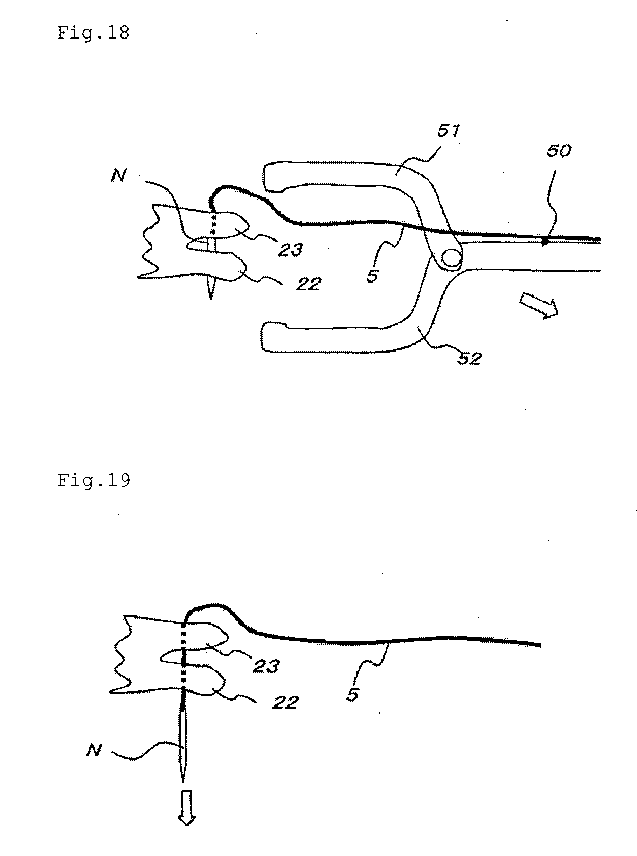

In Fig. 18, the fixed hook 52 is separated from the suture needle N and the movable hook type suture inserting device 50 is separated from the diseased portion.
In Fig. 19, the suture needle N, which passes through the articular capsule 23 and the articular labrum 22, is pulled downward, as shown, so that the articular labrum 22 and the articular capsule 23 are stitched and the articular labrum 22 and the articular capsule 23 are engaged with the anchor (not shown), thereby completing a suture process.

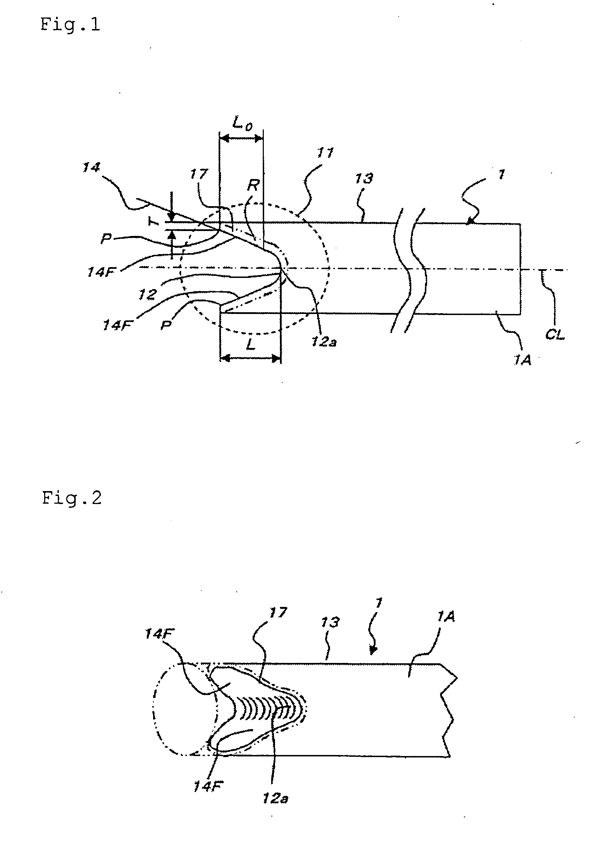

Meanwhile, when the suture thread 5 exists at the operating surgeon's side (near side) than the expected position, the suture thread 5 is tucked to the side remote from the operating surgeon (far side) using the device according to the first embodiment shown in Figs. 1 to 4.

Hereinafter, a configuration of a suture thread shifting device according to the first embodiment and a treatment method using the suture shifting device will be described.

The suture shifting device denoted by reference numeral 1 is obtained by machining a front edge 11 of a straight wire shaped member 1A, for example, based on a standard of a probe.

In the example shown in the figures, the suture thread shifting device 1 has a curved portion 12 having a predetermined radius R and a center point at a position shifted by a predetermined distance L from the side surface of the wire shaped member 1A having a circular section and from the front edge on a center line CL in a longitudinal direction of the material.

A slope surface 14F is formed along a straight line 14 which is in contact with the curved portion 12 and passes through a position P lower than a horizontal line 13 at a top of the member 1A having the circular section by a predetermined height T or a position remote from a deepest portion 12a of the curved portion 12 by the distance L. In other words, the front edge 11 is bisected.

The slope surface 14F and a boundary 17 of the wire shaped member 1A are formed of a smoothly curved surface by rounding edges, although not apparently shown in the figure.

Since the curved portion 12 catches and tucks the suture thread 5 (which is insoluble: a thread which insoluble for a long period is generally used in the surgery for the joint) to the far side, the radius R of the curved portion 12 is set to be equal to or larger than the radius of the section of the suture thread.

As described above, according to the present invention, the volume of the front edge does not increase as the pinch type device does, because the front edge of the wire is processed. Accordingly, the device hardly interferes with the bone or the like.
When the material device is too thin, workability deteriorates and thus the device may be curved. In addition, the device may pierce out of tissue like a needle.
Accordingly, it is preferable that the device has a material, which is to be processed, having rigidity by which deformation is not generated even when a general operation force is applied, such as stainless steel, and a diameter (for example, 2 to 5 mm) which prevents the device from unnecessarily passing through the tissue.
Meanwhile, when the device is too thick, since the device may interfere with the other thread or the tissue (the same problem as a pinch type device having the clip shaped member), an upper limit of the diameter must be considered.

The suture thread shifting device 1 is preferably straight. However, the suture thread shifting device may be bent or curved in a range that allows the thread to be tucked (shifted) to the far side through a minute incision (small hole) .

When the bisected inner sides, or the slope surfaces 14F, have irregularities, the suture thread may be damaged. When the suture thread is damaged, the strength of the thread deteriorates and a period for fixing the shoulder shortens. In addition, when the thread is tightened at the surgery, the thread may be broken.
In addition, when the surface of the device has irregularities, it becomes difficult to conduct the surgery because, for example, resistance against the human body tissue increases, and the human tissue may be damaged.
Accordingly, it is preferable that the bisected inner surfaces (slope surfaces 14F) are smoothly processed.

As the material of the device, a material which bears sterilization and has resistance against corrosion may be used. For the reason, stainless steel is preferably used.

When the length of the device is short, the device is not applicable to a patient whose deltoid muscle of the shoulder is developed in the surgery of shoulder, for example.
When the length of the device is long to some degree, the device may be additionally inserted even in a state where the other device is inserted into a joint.

When the length of the device is too long, it is difficult to operate the device. Accordingly, the length of the device is preferably in a range of 15 cm to 35 cm.

The suture thread shifting device according to the first embodiment needs not be a disposable device. In other words, the suture thread shifting device can be repeatedly used for a long time.

According to the suture thread shifting device of the first embodiment, a time for shifting the suture thread from a position on the operating surgeon's side (near side position) to a position remote from the operating surgeon (far side position) shortens to about a half.

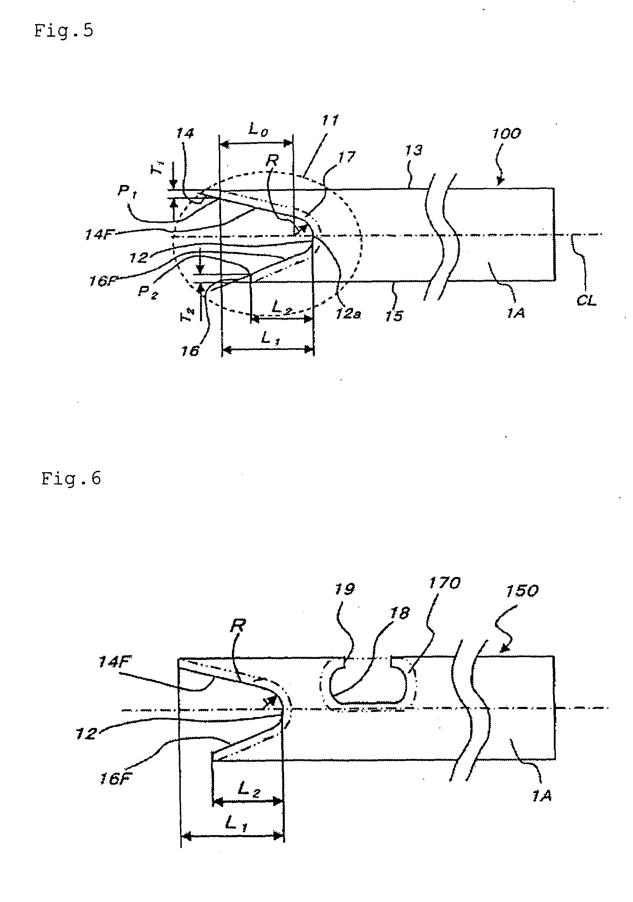

Next, a second embodiment will be described with reference to Fig. 5.
In the first embodiment shown in Figs. 1 to 4, the bisected portions of the front edge (portions denoted by the length L of Fig. 1) are bilaterally symmetrical. In contrast, in the second embodiment shown in Fig. 5, the bisected portions of the front edge are bilaterally asymmetrical.

The second embodiment will be described with reference to Fig. 5.
In the example shown in the figure, a suture thread shifting device 100 has a curved portion 12 having a predetermined radius R and a center at a position shifted by a predetermined distance L0 from the side surface of the wire shaped member 1A having a circular section and from the front edge on a center line CL in a longitudinal direction of the material.

A slope surface 14F is formed along a straight line 14 which is in contact with the curved portion 12, and passes through a position which is spaced from a deepest portion 12a of the curved portion by a distance L1 and is lower than a horizontal line 13 at a top of the member 1A having the circular section by a predetermined height T1.
Meanwhile, a slope surface 16F is formed along a straight line 16 which passes through a position which is spaced from the deepest portion 12a of the curved portion by a distance L2 (shorter than L1 in the example shown in the figure) and is higher than a horizontal line 15 at a bottom of the member 1A having the circular section.

The slope surfaces 14F and 16F and a boundary 17 of the wire shaped member 1A are smoothly curved surfaces by rounding edges, although not apparently shown.
The other configurations are similar to those of the first embodiment shown in Figs. 1 to 4 and thus their description will not be repeated.

According to the second embodiment having the above-described configuration, since the bisected portions (slope surfaces 14F and 16F), which are bilaterally asymmetrical, can catch (wind) or disengage the suture thread (release the wound thread) by rotating the suture thread shifting device 100, the usability of the suture thread shifting device 100 is excellent.

Next, a third embodiment will be described with reference to Fig. 6.
In the third embodiment shown in Fig. 6, an elliptical notch 18 formed at the rear side of the bisected portions (slope surfaces 14F and 16F) which are bilaterally asymmetrical is added to the embodiment shown in Fig. 5.
In addition, the notch 18 is connected to the outer circumference of a wire shaped member 1A by an opening 19. A boundary 170 between the notch 18 and the opening 19, and the wire shaped member 1A has a smooth surface by rounding corner edges.

When the notch 18 and the opening 19 are provided and the suture thread is engaged with the notch, it is possible to easily pull the suture thread to the operating surgeon' side, as in the case the above-described pinch type suture inserting device 40 (see Fig. 12) is utilized.

The above-described embodiments should be considered in descriptive sense only and not for purposes of limitation of a technical scope of the present invention.
For example, although, in the embodiments shown in the figures, the surgery for the dislocation is described, the present invention is widely applicable to the surgery using arthroscope or endoscope, such as the surgery for knee as well as the shoulder.

### Brief Description of the Drawings

Fig. 1 is a side view showing a structure of a first embodiment of the present invention.
Fig. 2 is a perspective view stereoscopically showing a suture thread shifting device according to the first embodiment of the present invention.
Fig. 3 is a state diagram showing a state before a suture thread is engaged when surgery is conducted using the first embodiment of the present invention.
Fig. 4 is a state diagram showing a state where a suture thread is engaged and pressed when the surgery is conducted using the first embodiment of the present invention.
Fig. 5 is a side view showing a structure of a second embodiment of the present invention.
Fig. 6 is a side view showing a structure of a third embodiment of the present invention.
Fig. 7 is a stereoscopic perspective view showing a structure of a human body around a shoulder joint.
Fig. 8 is a cross-sectional view of main portions of the shoulder joint.
Fig. 9 is a partial stereoscopic view showing an initial process of surgery for dislocation of a shoulder joint.
Fig. 10 is a partial stereoscopic view of a second step of the surgery for the dislocation of the shoulder joint.
Fig. 11 is a partial stereoscopic view of a third step of the surgery for the dislocation of the shoulder joint.
Fig. 12 is a stereoscopic view of a pinch type suture inserting device.
Fig. 13 is an enlarged view of a front edge of the pinch type suture inserting device.
Fig. 14 is an enlarged view of the front edge of the pinch type suture inserting device and shows a state where a suture thread is engaged.
Fig. 15 shows an initial step when surgery is conducted using a movable hook type suture inserting device.
Fig. 16 shows a second step when the surgery is conducted using the movable hook type suture inserting device.
Fig. 17 shows a third step when the surgery is conducted using the movable hook type suture inserting device.
Fig. 18 shows a fourth step when the surgery is conducted using the movable hook type suture inserting device.
Fig. 19 shows a fifth step when the surgery is conducted using the movable hook type suture inserting device.

### Explanation of Symbols

1, 100, 150: suture thread shifting device
1A: wire shaped member
2: shoulder blade
3: humerus
4: anchor
5: suture thread
7: tubular surgery needle/blitz suture retriever
8: wire loop
11: front edge
12: curved portion
14F, 16F: slope surface
17: boundary
21: cartilage
22: articular labrum
23: articular capsule
30: humerus end
31: spherical protrusion

## Claims

1. A suture thread shifting device, wherein even when an incision portion of a surface layer of a human body is small, a front edge of the device has a sectional configuration suitable for allowing the front edge to reach a region in arthroscopic or endoscopic viewing field and the device with the suture thread engaging in the front edge can shift the suture thread from a position on the operating surgeon's side to a position remote from the operating surgeon.

2. The suture thread shifting device according to Claim 1, wherein the front edge is bisected, the lengths of bisected portions are different from each other, and the surfaces of the bisected portions are smoothly curved.
